# EUROPÄISCHE PATENTSCHRIFT

(11) **EP 1 690 849 B1**
(45) Veröffentlichungstag und Bekanntmachung des Hinweises auf die Patenterteilung: **26.08.2009**
(21) Anmeldenummer: 06405007.3
(22) Anmeldetag: 11.01.2006
(51) Int. Cl.: C07C 67/08, C07C 69/14, C07C 69/02, B01J 19/00, B01D 3/00

(54) **Verfahren zur Herstellung von Carbonsäureester mittels einer Reaktivdestillation**
Process for preparing a carboxylic acid ester by reactif distillation
Procédé de préparation d'un ester d'une acide carboxylique par destillation réactive

(30) Priorität: 10.02.2005 EP 05405063
(43) Veröffentlichungstag der Anmeldung: 16.08.2006
(73) Patentinhaber: Sulzer Chemtech AG, 8404 Winterthur (CH)
(72) Erfinder: Zuber, Laurent, 8404 Winterthur (CH); Bailer, Oliver, 8404 Winterthur (CH); Sander, Stefan, 8408 Winterthur (CH); Meierhofer, Heinz, 8545 Rickenbach (CH)
(74) Vertreter: Sulzer Management AG

(56) Entgegenhaltungen:
- EP-A- 1 424 115
- WO-A-99/48855
- DE-A1- 19 829 809
- J. GMEHLING: "Grundoperationen" 1996, GEORG THIEME VERLAG , STUTTGART * Seite 221 *
- K. SATTLER: "Thermische Trennverfahren" 1988, VCH VERLAGSGESELLSCHAFT MBH , WEINHEIM * Seite 140 *

## Beschreibung

Die Erfindung betrifft ein Verfahren zur Herstellung von Carbonsäureester mittels einer Reaktivdestillation gemäss Oberbegriff von Anspruch 1. Der Carbonsäureester muss bei diesem Verfahren gegenüber den Edukten Carbonsäure und Alkohol höhersiedend sein. Die Erfindung betrifft insbesondere ein Verfahren zur Herstellung von Butylacetat oder Isobutylacetat, nämlich durch Veresterung von Essigsäure mit Butanol bzw. Isobutanol. Sie bezieht sich auch auf Anlagen zum Durchführen des erfindungsgemässen Verfahrens.

Das gattungsgemässe Veresterungs-Verfahren mit einer Reaktivdestillation ist aus der WO-A 99 48855 bekannt. Butylacetat oder Isobutylacetat wird als höhersiedendes Sumpfprodukt in einer Kolonne gewonnen, in der die reaktivdestillative Veresterung von Essigsäure mit n-Butanol bzw. Isobutanol katalytisch durchgeführt wird, wobei diese Katalyse in einer Reaktionszone zwischen zwei Trennzonen stattfindet. Im Folgenden wird unter dem Alkohol Butanol (BuOH) jeweils auch Isobutanol oder ein weiterer Alkohol (A) und entsprechend unter dem Ester Butylacetat (BuOAc) auch Isobutylacetat oder ein weiterer Ester (E) verstanden. Aus einem Kopfprodukt der Kolonne wird ein organischer Anteil (BuOH + BuOAc bzw. A + E) von Wasser (W) getrennt und in die obere Trennzone zurückgeführt. Dank den Trennzonen erscheint weitgehend keine Essigsäure (S) sowohl im Sumpfprodukt als auch im Kopfprodukt. Essigsäure, die nach oben und nach unten aus der Reaktionszone 2 austritt, wird dank den beiden Trennzonen in die Reaktionszone zurück transportiert, wo sie schliesslich vollständig oder zumindest angenähert vollständig umgesetzt wird. Essigsäure und unerwünschte Nebenprodukte erscheinen nur in geringen Mengen als Verunreinigungen im Butylacetat.

Statt Essigsäure CH₃-COOH kann auch Essigsäureanhydrid (CH₃-CO)₂O (oder eine Mischung) als Edukt eingesetzt werden. Essigsäureanhydrid geht mit Wasser - solches entsteht bei der Veresterung - in Essigsäure über.

Einem Hersteller von Butylacetat gelang es, bezüglich den in der WO-A 99 48855 beschriebenen Ausführungsformen des vorbekannten Verfahrens eine Verbesserung der Produktqualität zu erreichen, indem er durch Verwendung von besonderen Feststoffkatalysatoren die Verunreinigung des Butylacetats durch die unerwünschten Nebenprodukte (Dibutyläther) herabsetzen konnte. Allerdings führte diese Verbesserung dazu, dass im Produkt der Anteil an Essigsäure zunahm. Der Grund dafür ist anscheinend, dass die neu verwendeten Feststoffkatalysatoren nur bei Temperaturen stabil sind, die kleiner sind als bei den beschriebenen Beispielen des vorbekannten Verfahrens, und daher die Reaktivdestillation entsprechend bei einer tieferen Temperatur und folglich bei einem tieferen Druck - d.h. im Vakuumbetrieb - durchgeführt werden muss.

Die Essigsäure wird aus dem Sumpfprodukt in einer der Reaktivdestillation nachgeschalteten Teilanlage entfernt. Sie wird dabei mit einer Base gemäss einem Verfahren neutralisiert, das bereits von einem älteren Verfahren zur Herstellung von Butylacetat bekannt ist, bei dem ein flüssiger Katalysator statt dem Feststoffkatalysator verwendet worden ist (vgl. WO-A 99 48855, wo der ältere Stand der Technik gewürdigt wird). Die bei der Neutralisierung mit der Base gebildeten Salze werden durch eine Wäsche und eine Phasentrennung aus dem Butylacetat entfernt. Nachteil dieses Verfahrens ist, dass eine besondere Base zur Verfügung gestellt werden muss und dass eine Entsorgung der abgetrennten Salze, welche für die Umwelt eine starke Belastung bedeuten würden, kostspielig ist. Es wäre daher vorteilhaft, ohne die genannte Neutralisierung auskommen zu können.

Aufgabe der Erfindung ist es, eine weitere Verbesserung des Verfahrens zur Herstellung von Carbonsäureester zu schaffen, bei dem das Problem mit der Carbonsäure (insbesondere der Essigsäure), die in dem mittels der bekannten Reaktivdestillation erzeugten Carbonsäureester (Butylacetat) enthalten ist, ohne die Neutralisierung mit einer Base gelöst wird. Diese Aufgabe wird durch das im Anspruch 1 definierte Verfahren gelöst.

Das Verfahren zur Herstellung von Carbonsäureester (E) durch eine Veresterung wird mittels einer Reaktivdestillation in einer ersten Kolonne durchgeführt. Der Carbonsäureester ist gegenüber den Edukten Carbonsäure (S) und Alkohol (A) höhersiedend. Bei der Veresterung wird ein Feststoffkatalysator verwendet. Die Veresterung und eine destillative Trennung finden in drei Zonen statt, nämlich in einer Reaktionszone, einer oberen Trennzone sowie einer unteren Trennzone. Es werden ein erstes Kopfprodukt und ein erstes Sumpfprodukt erzeugt. Aus dem ersten Kopfprodukt wird eine wässrige Phase von einer organischen Phase getrennt. Wärme, die zum Antreiben der Reaktivdestillation dient, wird bei einer Temperatur zugeführt, für die sich in dem verdampften Sumpfprodukt ein Druck nicht grösser als Umgebungsdruck einstellt. Aus dem ersten Sumpfprodukt wird unter Entfernung eines Rests an Carbonsäure und Alkohol der Carbonsäureester gewonnen. Die Entfernung von Carbonsäure und Alkohol aus dem ersten Sumpfprodukt wird destillativ in einer zweiten Kolonne durchgeführt. Einem zweiten Sumpfprodukt, d.h. dem Sumpfprodukt der zweiten Kolonne, das weitgehend aus Carbonsäureester und geringen Mengen an weiteren Stoffen besteht, wird dabei Wärme bei einer Temperatur zugeführt, für die sich in dem verdampften Sumpfprodukt ein Druck grösser als Umgebungsdruck einstellt.

Bei der Analyse der Aufgabe nahm man zunächst an, dass im ersten Sumpfprodukt der Anteil der Essigsäure verringert werden kann, indem man die Anzahl der theoretischen Trennstufen in der unteren Trennzone vergrössert. Aus Erfahrung ist anzunehmen, dass die Trenneffekte bei Vakuumbedingungen besser als bei Umgebungsdruck sind. Es ist bekannt, dass mit zunehmendem Druck die relative Flüchtigkeit der Komponenten in einem Gemisch abnimmt, so dass das Gemisch bei erhöhtem Druck schlechter zu trennen ist. Die Methode der ersten Wahl, die destillative Trennung eines Gemisches zu erleichtern, besteht daher darin, die Trennaufgabe bei niedrigerem Druck durchzuführen, wie in EP 1 424 115 A1 beschrieben, insbesondere im Vakuum statt bei Umgebungsdruck. Ausgehend von diesen Überlegungen entschloss man sich zu untersuchen, ob eine Destillation bei niedrigerem Druck den erwünschten Trenneffekt tatsächlich ergibt. Aufgrund bestehender Betriebsdaten musste man jedoch schliessen, dass die Trennung unter dieser Bedingung nicht besser ist. Entgegen allgemein anerkannter Erfahrung wurde beschlossen, die Trennung bei höheren Drücken experimentell zu untersuchen.

Überraschenderweise ergaben sich nun Resultate, die nicht der Erfahrungsregel entsprachen: Die Trennung war bei erhöhtem Druck besser. Dieses Resultat führte zur erfindungsgemässen Lösung der Aufgabe: Die Destillation wird also in einer zweiten Kolonne und bei einem erhöhten Druck durchgeführt.

Die abhängigen Ansprüche 2 bis 8 betreffen vorteilhafte Ausführungsformen des erfindungsgemässen Verfahrens. Anlagen zur reaktivdestillativen Herstellung von Carbonsäureester sind Gegenstand der Ansprüche 9 bis 10.

Nachfolgend wird die Erfindung anhand der Zeichnungen erläutert. Es zeigen:
- Fig. 1: eine Anlage mit einer Kolonne, die bei der Durchführung des erfindungsgemässen Verfahrens verwendet wird,
- Fig. 2: einen Ausschnitt aus einer katalytischen Reaktionszone,
- Fig. 3: eine Teilanlage zur Reinigung des erzeugten Carbonsäureesters, wie sie das erfindungsgemässe Verfahren vorsieht, und
- Fig. 4: ein Blockbild zu einer speziellen Gesamtanlage, in der das erfindungsgemässe Verfahren zur Anwendung kommt.

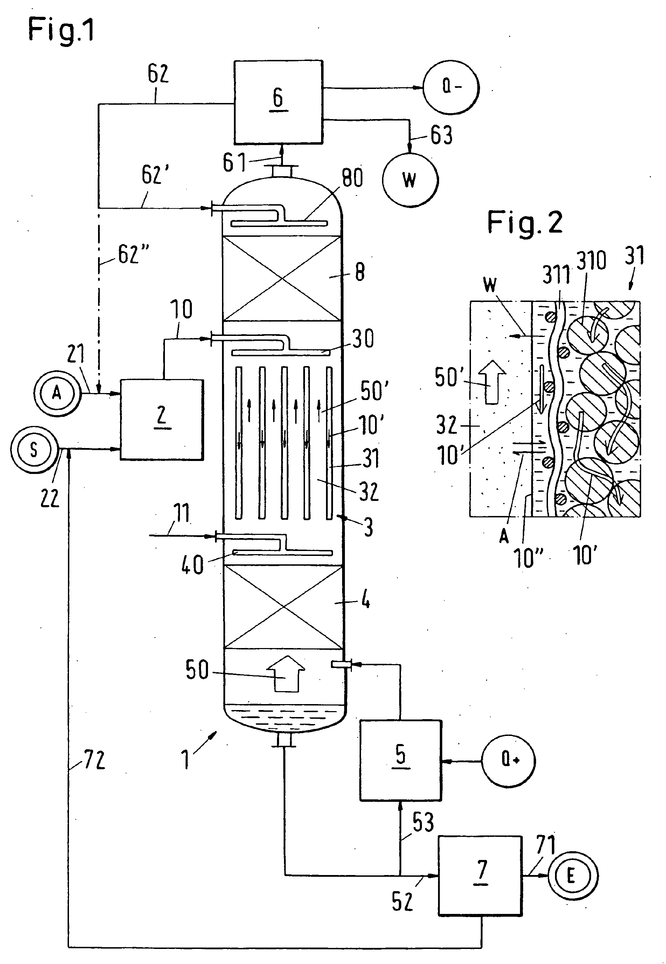

Die in Fig. 1 dargestellte Anlage umfasst eine erste Kolonne 1, in der eine reaktivdestillative Veresterung durchgeführt wird. Das Verfahren wird für die Herstellung von Butylacetat beschrieben. Gleiches gilt für andere Carbonsäureester. Aus deren Sumpf wird das Essigsäure enthaltende Butylacetat abgezogen. Durch eine Leitung 52 wird dieses Produkt, dessen Qualität noch ungenügend ist, einer Teilanlage 7 zur Reinigung zugeführt.

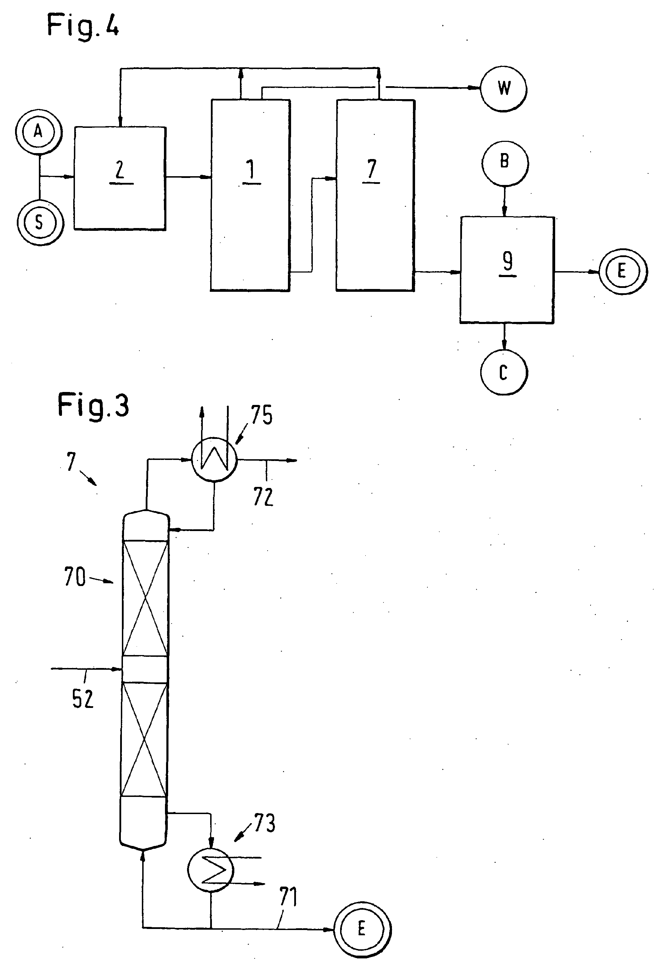

Die Einspeiseprodukte des erfindungsgemässen Verfahrens sind Butanol, nachfolgend als Alkohol A bezeichnet, und Essigsäure S. Das Sumpfprodukt der erste Kolonne 1, nachfolgend als erstes Sumpfprodukt bezeichnet, wird in der Teilanlage 7 und mit einer zweiten Kolonne 70 (vgl. Fig. 3) weiter aufbereitet, so dass schliesslich ein Butylacetat E (Ester) gewonnen werden kann, dessen Essigsäuregehalt beispielsweise unter 100 Gew-ppm liegt. Das gereinigte Produkt wird durch die Leitung 71 abgezogen. Ein in der zweiten Kolonne abgetrenntes Kopfprodukt, nämlich das zweite Kopfprodukt, wird über eine Leitung 72 in die Reaktivdestillation zurück geführt.

In der ersten Kolonne 1 werden die Essigsäure S (Strom 22) und der Alkohol A (Strom 21) in einem molaren Verhältnis im Bereich zwischen 5 : 1 und 1 : 10 eingespeist, vorzugsweise mit einem Überschuss an Alkohol A, der 100 % (molares Verhältnis 1 : 2) nicht übersteigt. Diese Einspeiseprodukte können getrennt oder in einer Mischung eingespeist werden.

Die Kolonne 1 enthält einen Packungsteil, der eine Reaktionszone 3 bildet und in welchem die Veresterung mittels einer heterogenen Katalyse durchgeführt wird. Durch eine Leitung 10 und mit einem Verteiler 30, werden Alkohol A und Essigsäure S (zusammen mit möglicherweise in einem Vorreaktor entstandenem oder durch Rückführung eingebrachtem, teilweise umgesetztem Produktgemisch) auf die Packung der Reaktionszone 3 aufgebracht. Unter dieser Packung ist ein Verteiler 40 und ein zweite Packung, die untere Trennzone 4, angeordnet. Eine Leitung 11, durch die zusätzlich Alkohol A eingespeist werden kann, und der Verteiler 40 sind optional.

Die aus der Reaktionszone 3 und der unteren Trennzone 4 bestehenden Einbauten wirken als Abtriebsteil. Es wird Wärme Q ₊ zum Antrieb der Reaktivdestillation benötigt. Im Sumpf wird durch eine Zufuhr dieser Wärme Q ₊ ein Dampfstrom 50 erzeugt, der Alkohol A, Essigsäure S und einen kleinen Anteil an Butylacetat enthält. Dieser Dampfstrom 50 bildet einen Gegenstrom zu einem auf der Packung fliessenden Rieselfilm und führt einerseits Essigsäure in die Reaktionszone zurück. Andererseits bildet er einen als Strippgas wirkenden Dampfstrom 50', mit dem Wasser W aus der Reaktionszone 3 entfernt wird, wodurch die Reaktionskinetik zugunsten der Esterbildung beeinflusst wird. Die für das Verdampfen benötigte Wärme Q₊ wird in einer Einrichtung 5 einem Zweigstrom 53 zugeführt. Bereits in der unteren Trennzone 4 wird aus dem Flüssigkeitsgemisch, das aus der Reaktionszone 3 austritt, Wasser W und ausserdem auch Alkohol A durch den Dampfstrom 50 aufgenommen.

Oberhalb der Reaktionszone 3 sind eine weitere Packung, nämlich die obere Trennzone 8, und ein Verteiler 80 in der ersten Kolonne 1 eingebaut. Der am Kopf austretende Dampfstrom (Pfeil 61) wird in einer Einrichtung 6 verflüssigt (Wärmeabfuhr Q₋) und dieses Kopfprodukt, nämlich das erste Kopfprodukt, in zwei Fraktionen 62 und 63 getrennt, beispielsweise durch Dekantieren. Die Fraktion 63 des ersten Kopfprodukts ist im wesentlichen flüssiges Wasser W, in dem organische Restanteile gelöst sind. Die Fraktion 62 ist eine flüssige organische Phase. Sie wird als Rücklauf 62' auf die obere Trennzone 8 zurück geführt. Mit der oberen Trennzone 8 wird Essigsäure S, die vom Dampfstrom 50 mitgeschleppt wird, abgefangen. Ein auf der Packung fliessender Rieselfilm transportiert die abgefangene Essigsäure wieder in die Reaktionszone 3 zurück.

Die organische Phase 62 aus dem ersten Kopfprodukt wird direkt (Rücklauf 62') oder indirekt (gestrichelt gezeichnete Verbindung 62") in die Reaktionszone 3 zurück geführt. Für das Massenverhältnis zwischen dem frisch eingespiesenen Alkohol A (Strom 21) und der zurückgeführten organischen Phase 62 werden Werte zwischen 5 : 1 bis 1 : 20 gewählt. Für den Rücklauf 62' wird 60 bis 100% der gesamten Menge an abgetrennter organischer Phase 62 verwendet.

Die katalytische Reaktionszone 3 setzt sich im gezeigten Beispiel aus vertikalen Lagen 31 zusammen, zwischen denen Strömungskanäle 32 für den als Strippgas dienenden Dampfstrom 50' liegen. In und auf den Lagen 31 fliesst ein Gemisch 10', das die Einspeisestoffe A und S enthält. Details zur Reaktionszone 3 sind im Ausschnitt der Fig. 2 dargestellt, wo eine Grenzregion der Lage 31 abgebildet ist; diese Grenzregion umfasst Teile eines Strömungskanals 32 mit dem Dampfstrom 50' sowie der Lage 31, die ein Granulat 310 eines Katalysators enthält. Ein Gewebe 311 aus einem beständigen Material bildet ein Gefäss, in dem das Granulat 310 eingeschlossen ist. Innerhalb des Gefässes und auf der äusseren Seite des Gewebes 311 fliesst das Gemisch 10', mit mäandrischen Strömungsfäden innerhalb und als Rieselfilm 10" ausserhalb des Gefässes. Durch die freie Oberfläche des Rieselfilms 10" tritt Wasser W in den Dampfstrom 50' über, da in diesem ein relativ kleiner Partialdruck des Wassers W vorliegt. Der Partialdruck des Alkohols A ist relativ gross, so dass für eine Abgabe und Aufnahme von Molekülen des Alkohols A an der Oberfläche des Rieselfilms 10" weitgehend ein Gleichgewicht besteht.

Die Reaktivdestillation wird unter Verwendung von Kolonneneinbauten durchgeführt, auf oder in denen der Katalysator fixiert ist und durch die ein guter Kontakt sowohl zwischen flüssiger Phase und Katalysator als auch zwischen flüssiger und gasförmiger Phase ermöglicht wird. Die als Trägerstruktur für den Katalysator dienenden Kolonneneinbauten sind unter Anwendung an sich bekannter Formen ausgebildet, wobei diese Kolonneneinbauten auch verschiedene Formen kombinieren können, nämlich eine Kombination von mindestens einem Destillationsboden und/oder mindestens einem strukturierten Packungselement, das aus zick-zack- oder wellenförmigen, eine Kreuzkanalstruktur bildenden Lagen zusammengesetzt ist, und/oder mindestens einer Zone aus Schüttfüllkörpern. Geeignete Packungen für die Reaktionszone 3 sind in der EP-A- 0 396 650 oder in der EP-A- 0 631 813 beschrieben. Andere bekannte Reaktivdestillations-Einrichtungen können für diesen Zweck verwendet werden, unter anderem auch Böden mit speziellen Einrichtungen (vgl. US-A- 5 914 011).

Als Katalysator lässt sich ein lonentauscherharz verwenden, wobei dieses vorzugsweise in stark saurer Form vorliegt und makroporös ausgebildet ist. Der Katalysator muss bei Temperaturen zwischen 80°C und 120°C verwendbar sein; in diesem Temperaturintervall darf er nur moderat an Aktivität einbüssen.

Mit Vorteil werden die Einspeisestoffe Alkohol A und Essigsäure S schon in einem Vorreaktor 2 bis zum Gleichgewicht zur Reaktion gebracht, wobei 10 bis 75% der Essigsäure S in Ester E umgesetzt wird. Durch die Leitung 10 tritt somit ein Gemisch aus Alkohol A, Essigsäure S, Ester E und Wasser W in die erste Kolonne 1 ein. In der Reaktionszone 3 wird dann die noch verbleibende Essigsäure S weiter umgesetzt, so dass ein Gesamtumsatz der in den Vorreaktor 2 eingespeisten Essigsäure von über 95% erreicht wird.

Die in der Teilanlage 7 - siehe Fig. 3 - erfindungsgemäss durchgeführte Entfernung von Essigsäure S und Alkohol A aus dem ersten Sumpfprodukt wird destillativ in einer zweiten Kolonne 70 auf deren Packung durchgeführt. Die Kolonne ist mit Stoffaustauscheinrichtungen gefüllt, z.B. Schüttfüllkörper, strukturierte Packungen oder Böden. Dabei wird Wärme (Heizapparat 73) einem zweiten Sumpfprodukt, d.h. dem Sumpfprodukt der zweiten Kolonne 70, bei einem Überdruck gegenüber der Umgebung zugeführt, wobei das zweite Sumpfprodukt weitgehend aus Ester E (beispielsweise Butylacetat oder Isobutylacetat) und geringen Mengen an weiteren Stoffen (beispielsweise 50 ppm Essigsäure) besteht.

Die Verfahrensbedingungen werden in der zweiten Kolonne 70 so vorgegeben, dass der Druck hinsichtlich einer destillativen Trennwirkung möglichst gross ist, nämlich dass der absolute Druck am unteren Rand der Einbauten mindestens 1.5 bar und vorzugsweise mehr als 2 bar beträgt - vorzugsweise möglichst um Einiges grösser als 2 bar. Da ein erhöhter Druck auch eine erhöhte Temperatur bedeutet, ist der Druck in der zweiten Kolonne 70 aufgrund von Korrosions- und Festigkeitssproblemen nach oben vorzugssweise auf 3 bar limitiert.

Ein zweites Kopfprodukt, d.h. das Destillat der zweiten Kolonne 70, wird nach einer Verflüssigung (in einem Kondenser 75) direkt - oder indirekt über den Vorreaktor 2 - in die erste Kolonne 1 eingespeist. Dies kann zusammen mit der Rückführung von organischer Phase 62 des ersten Kopfprodukts geschehen.

Es ist eine Alternative zum Neutralisieren der Essigsäure des ersten Sumpfprodukts gesucht und mit der erfindungsgemässen Lösung gefunden worden. Trotzdem kann es von Vorteil sein, wenn dem Sumpf der zweiten Kolonne 7 eine Neutralisierungsstufe 9 - siehe Fig. 4 - nachgeschaltet ist, mit der Reste von Essigsäure entfernt werden, wie dies bereits als letzte Stufe im zu verbessernden Verfahren getan worden ist. Die Fig. 4 zeigt in einem Blockbild eine spezielle Gesamtanlage, in der alle oben genannten Teilanlagen zusammengefasst sind: Vorreaktor 2, erste Kolonne 1, zweite Kolonne 7 und Neutralisierungsstufe 9. Das zweite Sumpfprodukt der zweiten Kolonne 7, in dem noch Reste von Essigsäure enthalten sind, wird mit einer Base B neutralisiert. Die dabei gebildeten Salze C werden mittels einer Wäsche sowie einer Phasentrennung aus dem Ester E entfernt. Die Salzfracht, die nicht ganz vermieden werden kann, ist jedoch gegenüber dem vorbekannten Verfahren deutlich reduziert; es fällt typischerweise 100 bis 500 Mal weniger Salz an. Der Salzverbrauch kann - in Moläquivalenten ausgedrückt - auf unter 0.05 mol-% reduziert werden Für die Produktion von 1'162 kg/h BuOAc (oder 10'000 mol/h) muss man beispielsweise statt 12 kg/h nur noch 0.12 kg/h Essigsäure neutralisieren. Der Salzverbrauch kann somit von 1.9 mol-% auf 0.019 mol-% reduziert werden.

## Patentansprüche

1. Verfahren zur Herstellung von Carbonsäureester (E), der gegenüber den Edukten Carbonsäure (S) und Alkohol (A) höhersiedend ist, mittels einer Reaktivdestillation in einer ersten Kolonne (1) und unter Verwendung eines Feststoffkatalysators, wobei
- eine Veresterung und eine destillative Trennung in drei Zonen - einer Reaktionszone (3), einer oberen Trennzone (8) sowie einer unteren Trennzone (4) - durchgeführt werden,
- ein erstes Kopfprodukt (61) und ein erstes Sumpfprodukt (52) erzeugt werden,
- aus dem ersten Kopfprodukt eine wässrige Phase (63) von einer organischen Phase (62) getrennt wird,
- Wärme (Q ₊), die zum Antrieb der Reaktivdestillation benötigt wird, bei einer Temperatur zugeführt wird, für die sich in dem verdampften Sumpfprodukt ein Druck nicht grösser als Umgebungsdruck einstellt, und
- aus dem ersten Sumpfprodukt unter Entfernung eines Rests an Carbonsäure und Alkohol der Carbonsäureester gewonnen wird,
**dadurch gekennzeichnet, dass**
die Entfernung von Carbonsäure und Alkohol aus dem ersten Sumpfprodukt destillativ in einer zweiten Kolonne (70) durchgeführt wird, wobei Wärme einem zweiten Sumpfprodukt (71), d.h. dem Sumpfprodukt der zweiten Kolonne, das weitgehend aus Carbonsäureester und geringen Mengen an weiteren Stoffen besteht, bei einer Temperatur zugeführt wird, für die sich in dem verdampften Sumpfprodukt ein Druck grösser als Umgebungsdruck einstellt.

2. Verfahren nach Anspruch 1, **dadurch gekennzeichnet, dass** der Carbonsäureester (E) Butylacetat oder Isobutylacetat, der Alkohol (A) Butanol bzw. Isobutanol und die Carbonsäure (S) Essigsäure ist.

3. Verfahren nach Anspruch 1 oder 2, **dadurch gekennzeichnet, dass** in der zweiten Kolonne (70) für die Destillation Einbauten angeordnet sind und dass der Druck hinsichtlich einer destillativen Trennwirkung möglichst gross ist, dass nämlich der absolute Druck am unteren Rand der Einbauten mindestens 1.5 bar und vorzugsweise mehr als 2 bar beträgt.

4. Verfahren nach einem der Ansprüche 1 bis 3, **dadurch gekennzeichnet, dass** ein zweites Kopfprodukt (72), d.h. ein Destillat der zweiten Kolonne, direkt - oder indirekt über einen Vorreaktor - in die erste Kolonne (1) eingespeist wird und dass dies gemeinsam mit einer Rückführung von organischer Phase (62) aus dem ersten Kopfprodukt (61) geschehen kann.

5. Verfahren nach einem der Ansprüche 1 bis 4, **dadurch gekennzeichnet, dass** in der ersten Kolonne (1) Carbonsäure (S) und Alkohol (A) in einem molaren Verhältnis von 5 : 1 bis 1 : 20, vorzugsweise 1 : 1 bis 1 : 2, getrennt oder in einer Mischung eingespeist werden, wobei zusätzlich auch Ester (E) und/oder Wasser (W) miteingespeist werden können, und dass insbesondere die Carbonsäure Essigsäure, Essigsäureanhydrid oder ein Gemisch dieser Stoffe ist.

6. Verfahren nach einem der Ansprüche 1 bis 5, **dadurch gekennzeichnet, dass** organische Phase (62) aus dem ersten Kopfprodukt (61) direkt oder indirekt in die Reaktionszone (3) zurückgeführt wird, dass für das Mengenverhältnis zwischen dem frisch eingespiesenen Alkohol (A, 21) und der zurückgeführten organischen Phase Werte zwischen 5 : 1 bis 1 : 20 gewählt werden und dass für den Rücklauf 60 bis 100% der gesamten Menge an abgetrennter organischer Phase verwendet wird.

7. Verfahren nach einem der Ansprüche 1 bis 6, **dadurch gekennzeichnet, dass** die Reaktivdestillation unter Verwendung von Kolonneneinbauten (31) durchgeführt wird, auf denen oder in denen der Katalysator fixiert ist und durch die sich ein guter Kontakt sowohl zwischen flüssiger Phase (10') und Katalysator als auch zwischen flüssiger und gasförmiger Phase (50') ergibt, wobei diese Phasen sich im Gegenstrom durch die Kolonneneinbauten bewegen.

8. Verfahren nach Anspruch 7, **dadurch gekennzeichnet, dass** die als Trägerstruktur für den Katalysator dienenden Kolonneneinbauten (31) als eine Kombination von mindestens einem Destillationsboden und/oder mindestens einer Zone von Schüttfüllkörpern ausgebildet ist und/oder mindestens einem strukturierten Packungselement, das aus zick-zack- oder wellenförmigen, eine Kreuzkanalstruktur bildenden Lagen zusammengesetzt ist.

9. Anlage zur reaktivdestillativen Herstellung von Carbonsäureester mit dem Verfahren gemäss einem der Ansprüche 1 bis 8, wobei der ersten Kolonne (1) ein Vorreaktor (2) vorgeschaltet ist, während die zweite Kolonne (70) nachgeschaltet ist, wobei- im Vorreaktor eine erste Stufe der Veresterung ohne Stofftrennung durchführbar ist, **dadurch gekennzeichnet, dass** ein Eingang des Vorreaktors durch eine Rückführleitung (62") für organische Phase des ersten Kopfprodukts (61) mit der ersten Kolonne und/oder durch eine Rückführleitung (72) mit der zweiten Kolonne verbunden ist, welche das zweite Kopfprodukt als Destillat enthält, wobei die erste Kolonne (1) für einen Vakuumbetrieb und die zweite Kolonne (70) für einen Überdruckbetrieb bei einem absoluten Druck am unteren Rand der Einbauten von mindestens 1,5 bar und für einen maximalen Innendruck von 5 bar absolut ausgelegt sind.

10. Anlage nach Anspruch 9, **dadurch gekennzeichnet, dass** dem Sumpf der zweiten Kolonne (70) eine Teilanlage (9) nachgeschaltet ist, in der Reste von Carbonsäure mit einer Base neutralisierbar und die dabei gebildeten Salze mittels einer Wäsche und einer Phasentrennung aus dem Ester entfernbar sind, wobei als Base eine wässrige Lösung einer chemischen Verbindung verwendbar ist, die höchstens in Spuren eine Verseifung mit dem Ester zur Folge hat, und der Salzverbrauch auf unter 0.05 mol-% reduziert ist.

## Claims

1. A method for the manufacture of carboxylic acid ester (E) which boils at a higher temperature than the educts carboxylic acid (S) and alcohol (A), by means of a reactive distillation in a first column (1) and using a solid material catalyst, wherein
- an esterification and a distillative separation in three zones - a reaction zone (3), an upper separating zone (8) and also a lower separating zone (4) - are carried out,
- a first head product (61) and a first sump product (52) are produced,
- an aqueous phase (63) is separated from an organic phase (62) from the first head product,
- heat (Q₊) which is required to drive the reactive distillation, is supplied at a temperature for which a pressure not greater than the ambient pressure sets in in the evaporated sump product, and
- the carboxylic acid ester is obtained from the first sump product with the removal of a residue of carboxylic acid and alcohol,
**characterised in that**
the removal of carboxylic acid and alcohol from the first sump produced is carried out by distillation in a second column (70), with heat being supplied to a second sump product (71) i.e. to the sump product of the second column, which largely comprises carboxylic acid ester and small quantities of further materials, at a temperature for which a pressure greater than the ambient pressure sets in in the evaporated sump product.

2. A method in accordance with claim 1, **characterised in that** the carboxylic acid ester (E) is butylacetate or isobutylacetate, the alcohol (A) is butanol and/or isobutanol and the carboxylic acid (S) is acetic acid.

3. A method in accordance with claim 1 or claim 2, **characterised in that** inserts are arranged in the second column (70) for the distillation and **in that** the pressure is as large as possible with respect to a distillative separation effect, **in that** the absolute pressure at the lower edge of the inserts namely amounts to at least 1.5 bar and preferably to more than 2 bar.

4. A method in accordance with any one of the claims 1 to 3, **characterised in that** a second head product (72), i.e. a distillate of the second column, is fed directly - or indirectly via a pre-reactor - into the first column (1) and **in that** this can take place together with a return of organic phase (62) from of the first head product (61).

5. A method in accordance with any one of the claims 1 to 4, **characterised in that** carboxylic acid (S) and alcohol (A) in a molar ratio of 5:1 to 1:20, preferably 1:1 to 1:2, are fed in separately or in a mixture in the first column (1), with it also being possible to feed in ester (E) and/or water (W) and **in that**, in particular, the carboxylic acid is acetic acid, acetic acid anhydride or a mixture of these substances.

6. A method in accordance with any one of the claims 1 to 5 **characterised in that** organic phase (62) from the first head product (61) is fed back directly or indirectly into the reaction zone, **in that** values between 5 : 1 to 1 : 20 are selected for the quantity ratio between the freshly fed in alcohol (A, 21) and the returned organic phase and **in that** 60% to 100% of the total amount of separated organic phase is used for the return.

7. A method in accordance with any one of the claims 1 to 6 **characterised in that** the reactive distillation is carried out using column inserts (31) on which or in which the catalyst is fixed and through which a good contact is made both between the liquid phase (10') and the catalyst and also between the liquid and gaseous phase (50'), with these phases moving in counterflow through the column inserts.

8. A method in accordance with claim 7 **characterised in that** the column inserts (31) serving as the support structure for the catalyst is assembled as a combination of at least one distillation base and/or at least one zone of bulk filled bodies and/or at least one structured packing element which is composed of zigzag layers or corrugated layers forming a crossed channel structure.

9. A plant for the manufacture of carboxylic acid ester by reactive distillation using the method in accordance with any one of the claims 1 to 8, wherein a pre-reactor (2) is upstream of the first column (1) while the second column (70) is downstream, and wherein a first stage of the esterification without material separation can be carried out in the pre-reactor, **characterised in that** one inlet of the pre-reactor is connected by a return line (62") for organic phases of the first head product (61) to the first column and/or by a return line (72) to the second column which contains the second head product as distillate, wherein the first column (1) is designed for a vacuum operation and the second column (70) for an overpressure operation at an absolute pressure at the lower edge of the inserts of at least 1.5 bar and for a maximum internal pressure of 5 bar absolute.

10. A plant in accordance with claim 9, **characterised in that** the a plant part (9) is positioned downstream of the sump of the second column (70), in which remains of carboxylic acid can be neutralised with a base and the salts which are formed in this process can be removed from the ester by means of a wash and a phase separation, wherein an aqueous solution of a chemical compound can be used as the base which results at the most in traces of a saponification with the ester and with the consumption of salt being reduced to less than 0.05 mol-%.

## Revendications

1. Procédé de préparation d'ester d'acide carboxylique (E) qui, en comparaison des composés de départ acide carboxylique (S) et alcool (A), a un point d'ébullition plus élevé, au moyen d'une distillation réactive dans une première colonne (1) et moyennant l'utilisation d'un catalyseur solide, dans lequel
- une estérification et une séparation par distillation dans trois zones - une zone de réaction (3), une zone de séparation supérieure (8), ainsi qu'une zone de séparation inférieure (4) - sont effectuées,
- un premier produit de tête (61) et un premier produit de fond (52) sont obtenus,
- du premier produit de tête, une phase aqueuse (63) est séparée d'une phase organique (62),
- la chaleur (Q+) qui est nécessaire à promouvoir la distillation réactive est introduite à une température à laquelle s'établit dans le produit de fond évaporé une pression qui n'est pas plus grande que la pression ambiante et
- l'ester d'acide carboxylique est obtenu à partir du premier produit de fond après l'élimination d'un reste d'acide carboxylique et d'alcool,
**caractérisé en ce que**
l'élimination de l'acide carboxylique et de l'alcool depuis le premier produit de fond est effectuée par distillation dans une deuxième colonne (70), lors de quoi la chaleur est amenée à un deuxième produit de fond (71), c'est-à-dire au produit de fond de la deuxième colonne qui consiste essentiellement en ester d'acide carboxylique et en petites quantités d'autres substances, à une température à laquelle s'établit dans le produit de fond évaporé une pression qui est plus grande que la pression ambiante.

2. Procédé selon la revendication 1, **caractérisé en ce que** l'ester d'acide carboxylique (E) est l'acétate de butyle ou l'acétate d'isobutyle, l'alcool (A) est le butanol, respectivement l'isobutanol, et l'acide carboxylique est l'acide acétique.

3. Procédé selon la revendication 1 ou 2, **caractérisé en ce que** dans la deuxième colonne (70), des garnissages sont disposés pour la distillation et **en ce que** la pression, du point de vue d'une efficacité de séparation par distillation, est la plus grande possible, en l'occurrence **en ce que** la pression absolue au bord inférieur ses garnissages est d'au moins 1,5 bar et est de préférence de plus de 2 bars.

4. Procédé selon l'une quelconque des revendications 1 à 3, **caractérisé en ce qu'**un deuxième produit de tête (72), c'est-à-dire un distillat de la deuxième colonne, est introduit directement - ou indirectement par l'intermédiaire d'un pré-réacteur - dans la première colonne (1) et **en ce que** cela peut s'effectuer en commun avec un recyclage de phase organique (62) provenant du premier produit de tête (61).

5. Procédé selon l'une quelconque des revendications 1 à 4, **caractérisé en ce que** de l'acide carboxylique (S) et de l'alcool (A) sont introduits dans la première colonne (1), dans un rapport molaire de 5: 1 à 1: 20, de préférence de 1: 1 à 1: 2, séparément ou en un mélange, de l'ester (E) et/ou de l'eau (W) pouvant être aussi introduits en plus, et **en ce qu'**en particulier l'acide carboxylique est de l'acide acétique, de l'anhydride d'acide acétique ou un mélange de ces deux substances.

6. Procédé selon l'une quelconque des revendications 1 à 5, **caractérisé en ce que** la phase organique (62) provenant du premier produit de tête (61) est recyclée directement ou indirectement dans la zone de réaction (3), **en ce que** pour le rapport quantitatif entre l'alcool (A, 21) fraîchement introduit et la phase organique recyclée sont choisies des valeurs entre 5: 1 et 1: 20 et **en ce que** pour le reflux, 60 à 100 % de la quantité totale de phase organique séparée sont utilisés.

7. Procédé selon l'une quelconque des revendications 1 à 6, **caractérisé en ce que** la distillation réactive est exécutée moyennant l'utilisation de garnissages de colonne (31) sur lesquels ou dans lesquels le catalyseur est fixé et par lesquels il résulte un bon contact aussi bien entre la phase liquide (10') et le catalyseur qu'entre la phase liquide et la phase gazeuse (50'), ces phases se déplaçant à travers les garnissages de la colonne à contre-courant.

8. Procédé selon la revendication 7, **caractérisé en ce que** les garnissages de la colonne (31), servant de structure porteuse pour le catalyseur, sont formés comme combinaison d'au moins un plateau de distillation et/ou d'au moins une zone de corps de garnissage en vrac et/ou d'au moins un élément de garnissage structuré qui est composé de couches en zigzag ou ondulées formant une structure de canaux croisés.

9. Installation pour la préparation par distillation réactive d'ester d'acide carboxylique avec le procédé selon l'une quelconque des revendications 1 à 8, dans laquelle un pré-réacteur (2) est installé en amont de la première colonne (1) tandis que la deuxième colonne (70) est installée en aval, sachant que dans le pré-réacteur peut être effectuée une première étape de l'estérification sans séparation de matière, **caractérisée en ce qu'**une entrée du pré-réacteur est reliée à la première colonne par une conduite de recyclage (62") pour la phase organique du premier produit de tête (61) et/ou à la deuxième colonne par une conduite de recyclage (72), laquelle contient le deuxième produit de tête comme distillat, la première colonne (1) étant dimensionnée pour un fonctionnement sous vide et la deuxième colonne (70) pour un fonctionnement en surpression avec une pression absolue au bord inférieur des garnissages d'au moins 1,5 bar et pour une pression intérieure maximale de 5 bars absolus.

10. Installation selon la revendication 9, **caractérisée en ce qu'**une installation partielle (9) est ajoutée en aval du fond de la deuxième colonne (70), installation dans laquelle les restes d'acide carboxylique peuvent être neutralisés par une base et dans laquelle les sels ainsi formés peuvent être éliminés de l'ester au moyen d'un lavage et d'une séparation de phase, sachant que, comme base, on peut utiliser une solution aqueuse d'un composé chimique qui ne produit une saponification avec l'ester qu'au plus à l'échelle de traces et que la consommation en sel est réduite à moins de 0,05 % en mole.
